# EUROPÄISCHE PATENTSCHRIFT

(11) **EP 1 235 066 B1**
(45) Veröffentlichungstag und Bekanntmachung des Hinweises auf die Patenterteilung: **01.04.2009**
(21) Anmeldenummer: 01110399.1
(22) Anmeldetag: 27.04.2001
(51) Int. Cl.: G01N 21/35

(54) **Verfahren zur analytischen Untersuchung einer Bierprobe**
Process for analysing a beer sample
Procédé pour l'analyse d'un échantillon de bière

(30) Priorität: 23.02.2001 DE 10108712
(43) Veröffentlichungstag der Anmeldung: 28.08.2002
(73) Patentinhaber: Warsteiner Brauerei Haus Cramer KG, 59581 Warstein (DE)
(72) Erfinder: Bollmann, Heinrich Jörg, 44532 Lünen (DE)
(74) Vertreter: Basfeld, Rainer

(56) Entgegenhaltungen:
- WO-A-89/09931
- WO-A2-00/41502
- US-A- 3 923 399
- US-A- 5 697 373
- US-A- 5 750 995
- LAI YOKE W.: 'Potential of Fourier transform Infrared Spectroscopy for the authentication of vegetable oils' J.AGRIC.FOOD CHEM. Bd. 42, 1994, Seiten 1154 - 1159
- MUSTAFA ÖZILGEN: "Construction of quality control charts with sub-optimal size samples" FOOD CONTROL, Bd. 9, Nr. 1, 1998, Seiten 57-60,

## Beschreibung

Die vorliegende Erfindung betrifft ein Verfahren zur analytischen Untersuchung einer Bierprobe zwecks Feststellung, ob es sich um Bier einer bestimmten vorgegebenen Biermarke beziehungsweise Biersorte einer Biermarke handelt, bei dem man zunächst durch spektroskopische Messungen einer Probenreihe verschiedener Proben mit jeweils mehreren Messwerten für jede Probe eine als Referenz dienende Datenmatrix erzeugt, die als Modell dient, und danach entsprechende Messwerte von einer zu untersuchenden Fremdprobe spektroskopisch erstellt werden und dann durch Vergleich geprüft wird, ob die Messwerte der Fremdprobe innerhalb des zuvor gewonnenen Modells liegen, wobei die als Modell dienende Datenmatrix beim Hersteller durch spektroskopische Messungen einer Probenreihe verschiedener Proben einer vorgegebenen Biermarke erzeugt wird.

Hintergrund der vorliegenden Erfindung ist die Tatsache, dass Bier auf dem Weg von der Brauerei zum Endverbraucher mitunter Einflüssen ausgesetzt ist, die auf die Inhaltsstoffe des Bieres einwirken und sie je nach Intensität in unterschiedlichem Maße beeinflussen. Durch diese Beeinträchtigung der Qualität kann bei dem Endverbraucher beziehungsweise dem Brauereikunden der Eindruck entstehen, es handele sich tatsächlich nicht um das Bier derjenigen Biermarke, unter der es verkauft wird, da der Kunde den ihm geläufigen Geschmack dieser Biermarke nicht wiederfindet. Hier ist zu bedenken, dass es tatsächlich in nicht unerheblichem Umfang bei Markenbieren zu Missbrauchs- und Betrugsfällen kommt, sei es, dass Großhändler billigere Biere minderer Qualität in Fässer von Markenbieren abfüllen lassen, sei es, dass Gastwirte Fässer mit solchem Bier minderer Qualität anstechen, aber am Zapfhahn gegenüber dem Kunden dieses Bier als Markenbier ausgeben. Beide Fälle kommen in der Praxis vor in einem bislang nicht erfassbaren Umfang, da entsprechende Kontrollmechanismen fehlen. Diese Fälle führen beim Biertrinker zu erheblichen Irritationen, da er das getrunkene Bier minderer Qualität, welches als Markenbier ausgegeben wird, der Biermarke zuordnet, was bei den Herstellern von Markenbieren zu erheblichen Imageschäden führt. Folglich besteht ein hohes Interesse daran, ein Verfahren zu entwickeln, welches eine analytische Untersuchung einer Bierprobe mit vertretbarem Aufwand dort zulässt, wo das Bier gezapft wird, z. B. in einer Gaststätte.

Nach dem allgemeinen Stand der Brauerei-Labor-Technik werden solche Rückschlüsse über die traditionelle Bieranalytik gezogen, indem das Profil der analytischen Parameter eines Bieres beurteilt wird. Diese "Mustererkennung" erfolgt subjektiv, was beim Beurteiler entsprechende analytische Erfahrungen voraussetzt.

Als analytische Hauptparameter gelten Werte wie: Stammwürze, Restextrakt, Alkoholgehalt, pH-Wert, Leitwert, Farbe, Bitterwert, Gerbstoffgehalt, Aromaprofil der Gärungsnebenprodukte (Alkohole, Ester, Aldehyde, Vicinale Diketone, Schwefelverbindungen), Verteilung der Anionen (Chlorid, Sulfat, Phosphat, Nitrat), Sensorische Analytik durch geschulte und geübte Verkoster etc.

Zur Bestimmung analytischer Hauptparameter sind unterschiedliche Analysengänge, Analysengeräte und die Einhaltung entsprechender Umgebungsbedingungen notwendig. Für die Einsatzaufgabe vor Ort ist bei traditioneller Arbeitsweise letztlich ein fahrbares, mehr oder minder ausgestattetes, Brauereinlabor angezeigt, das über die notwendigen Analysengeräte, aber auch über Einbaumöbel, Medienversorgung (Gase, Strom und Stromerzeuger, Wasser und Abwasser), Klimatisierung und die vorgeschriebenen Schutzeinrichtungen verfügt. Ein derartiges Laborequipment auf einem Fahrzeug zu installieren bedeutet einen ganz erheblichen technischen und finanziellen Aufwand, dessen Verhältnismäßigkeit zur Einsatzaufgabe fraglich wird.

Hinzu kommt, dass Biere aus reinen Naturprodukten hergestellt werden, die - wie alle Naturprodukte - natürlichen Schwankungen unterliegen. Darüber hinaus sorgen Nuancierungen in den technologischen Prozessen für gewollte oder ungewollte Schwankungen in den Inhaltsstoffen. Dazu kommen Matrixeinflüsse und die zufälligen Fehler in den Analysen selbst, die für weitere Streuungen in den Werten sorgen und unter den Bedingungen eines mobilen Labors sicher nicht geringer werden. Aus dieser Kenntnis heraus ist die Suche nach dem spezifischen Fingerprint eines Bieres illusorisch. Das "Standardbier" gibt es nicht ohne Schwankungen in den analytischen Profilen.

Die bisher verfügbaren Analysemethoden, die herstellerseitig in den Labors der Brauereien angewandt werden, ermöglichen zwar die Untersuchung einzelner Qualitätsmerkmale wie z. B. Farbe, Geruch, Anteil an Bitterstoffen etc. Diese Methoden sind herstellerseitig dazu vorgesehen, eine Produktion gleichbleibender Qualität unabhängig von Einflüssen wie z. B. Schwankungen in der Qualität der Ausgangsstoffe, jahreszeitlich bedingten Witterungseinflüssen etc. sicherzustellen. Die meisten gängigen Qualitätsprüfungsmethoden, die hierbei angewandt werden, haben eine gewisse subjektive Komponente. Alle diese Methoden eignen sich jedoch nicht, um sicher festzustellen, ob es sich bei Bier aus einer bestimmten Bierprobe um Bier einer vorgegebenen Biermarke handelt oder gegebenenfalls ein Fremdbier. Dies rührt daher, dass am Markt eine Vielzahl von Biermarken erhältlich sind, was solche Untersuchungen erschwert und außerdem die Geschmackskomponenten, die charakteristische Merkmale einer individuellen Biermarke ausmachen, sehr komplex sind und sich nur schwer durch die bekannten Analysemethoden quantitativ oder qualitativ erfassen lassen. Rückschlüsse auf die Identität einer Bierprobe mittels der herkömmlichen Brauerei-Laboranalytik sind zudem subjektiv, selbst wenn sie auf der individuellen Erfahrung einer die Probe untersuchenden geübten Person beruhen.

Hinzu kommt noch der Nachteil, dass die gängigen Analysemethoden die in den Labors der Brauereien zur Überprüfung der gleichbleibenden Bierqualität angewandt werden mit einem erheblichen apparativen Aufwand verbunden sind, so dass ein mobiles Labor, welches derartige Untersuchungen vor Ort z. B. in einer Gaststätte durchführen könnte wegen des zu großen Aufwands nicht wirtschaftlich realisierbar wäre.

Die internationale Patentanmeldung WO 00/41502 A1 offenbart eine transportable Analysevorrichtung, mittels derer die Hauptbestandteile einer Produktprobe (beispielsweise einer Bierprobe) sowie die relativen Mengenverhältnisse dieser Hauptbestandteile analysiert werden können, um die Produktprobe authentifizieren zu können. Die Analysevorrichtung umfasst eine Lichtquelle zur Bestrahlung der Produktprobe mit elektromagnetischer Strahlung, einen optischen Detektor zur Detektion des von der bestrahlten Produktprobe emittierten Lichts und einen Kontrollschaltkreis zur Bestimmung der Authentizität oder Qualität der Produktprobe.

Aus der internationalen Patentanmeldung WO 89/09931 A1 ist ein Verfahren zur analytischen Untersuchung von Bierprodukten bekannt, bei dem Kontrollproben bekannter Bierprodukte durch spektrographische Analysen untersucht werden und außerdem entsprechende Analysen unbekannter Bierprodukte vorgenommen werden, um Fremdstoffe in der untersuchten Probe nachweisen zu können. Das aus der vorstehend genannten Druckschrift bekannte Verfahren dient lediglich der Überprüfung, ob eine Bierprobe kontaminiert ist. Mit anderen Worten wird also untersucht, ob die Bierprobe unerwünschte Fremdstoffe enthält. Das Verfahren wird beim Hersteller unmittelbar während des Abfüllprozesses des Bierprodukts durchgeführt, um auf diese Weise zu verhindern, dass die Fremdstoffe zusammen mit dem Bierprodukt in die entsprechenden Getränkebehälter gelangen. Die Untersuchung des Bierprodukts findet also ausschließlich beim Hersteller während des Herstell- beziehungsweise Abfüllprozesses statt. Eine Überprüfung am Ort des Konsums, ob es sich bei dem Bier um ein Bier einer bestimmten vorgegebenen Biermarke handelt, ist mit Hilfe dieses Verfahrens nicht möglich.

Die Aufgabe der vorliegenden Erfindung ist es ein Verfahren zur analytischen Untersuchung einer Bierprobe zur Verfügung zu stellen, welches es ermöglicht, festzustellen, ob es sich um Bier einer bestimmten vorgegebenen Biermarke handelt und welches mit vertretbarem Aufwand vor Ort, zum Beispiel dort, wo das Bier gezapft wird, durchführbar ist.

Die Lösung dieser Aufgabe liefert ein erfindungsgemäßes Verfahren zur analytischen Untersuchung einer Bierprobe der genannten Art mit den kennzeichnenden Merkmalen des Anspruchs 1. Das erfindungsgemäße Verfahren sieht vor, dass man zunächst durch spektroskopische Messungen einer Probenreihe verschiedener Proben der vorgegebenen Biermarke mit jeweils mehreren Messwerten für jede Probe eine als Referenz dienende Datenmatrix erzeugt, die als Modell dient, und danach entsprechende Messwerte von der zu untersuchenden Fremdprobe spektroskopisch erstellt werden und dann durch Vergleich geprüft wird, ob die Messwerte der Fremdprobe innerhalb des zuvor gewonnenen Modells liegen, wobei die als Modell dienende Datenmatrix beim Hersteller durch spektroskopische Messungen einer Probenreihe verschiedener Proben einer vorgegebenen Biermarke erzeugt wird. Das Verfahren zeichnet sich erfindungsgemäß dadurch aus, dass die zunächst als multivariater Datensatz vorliegende Datenmatrix durch Hauptkomponentenanalyse (PCA) in einen reduzierten Datensatz überführt wird, der als Modell für die zu vergleichende Fremdprobe dient, und dass die Fremdprobe mittels einer transportablen Analysevorrichtung vor Ort an einer Zapfstelle untersucht wird, um festzustellen, ob es sich bei dieser um Bier einer bestimmten vorgegebenen Biermarke handelt, für die zuvor das Modell erstellt wurde.

Die spektroskopischen Messungen der Probenreihe verschiedener Proben der vorgegebenen Biermarke mit jeweils mehreren Messwerten für jede Probe werden somit beim Hersteller der vorgegebenen Biermarke durchgeführt , so dass dieser sich unter Zuhilfenahme mathematisch statistischer Methoden, vorzugsweise unterstützt durch eine elektronische Datenverarbeitung, das seiner Biermarke entsprechende Modell erzeugt. Vor Ort an der Zapfstelle erfolgt dann nur die Messung der Fremdprobe, was mit vertretbarem Aufwand durch ein transportables Spektrometer erfolgen kann. Durch den anschließenden Vergleich wird festgestellt, ob der beziehungsweise die Messwerte für die Fremdprobe innerhalb des zuvor erstellten Modells liegen. Ist dies der Fall, kann daraus der Schluss gezogen werden, dass es sich dabei um Bier der vorgegebenen Biermarke handelt. Liegen die Messwerte außerhalb des Modells lässt dies den Schluss zu, dass es sich nicht um Bier der vorgegebenen Biermarke sondern um ein Fremdbier handelt. Diese Erkenntnis ist für den Markenhersteller unter den eingangs genannten Gesichtspunkten völlig ausreichend. Es ist nicht notwendig, das Fremdbier als Bier einer bestimmten Biermarke zu identifizieren.

Allerdings kann der Inhaber des Biers der vorgegebenen Biermarke natürlich auch Modelle anderer Biermarken von Wettbewerbern erstellen, so dass die untersuchte verdächtige Fremdprobe gegebenenfalls einem dieser Modelle für eine der vorher durchgemessenen Biermarken zugeordnet werden kann. Ziel ist es im Rahmen des vorliegenden Verfahrens jedoch in der Regel nur, zu überprüfen, ob die untersuchte Bierprobe der vorgegebenen Biermarke des in der Regel die Untersuchung veranlassenden Markenherstellers entspricht.

Vorzugsweise wählt man gemäß einer Weiterbildung des erfindungsgemäßen Verfahrens als spektroskopische Messung die Messung der Transmission oder Absorption der Probe, wobei man vorzugsweise mindestens teilweise im Infrarotbereich misst. Weiter vorzugsweise misst man die Transmission oder Absorption der Probe im nahen Infrarotbereich und im sichtbaren Bereich.

Eine mögliche bevorzugte Weiterbildung des erfindungsgemäßen Verfahrens sieht vor, dass die als Referenz dienende Datenmatrix durch Messung der Transmission oder Absorption bei einer größeren Anzahl unterschiedlicher Wellenlängen für jeweils eine größere Anzahl verschiedener Proben der vorgegebenen Biermarke erzeugt wird. Man erhält auf diese Weise für jede der gemessenen Bierproben eine Mehrzahl, vorzugsweise eine Vielzahl von Messpunkten bei unterschiedlichen Wellenlängen. Diese Messpunkte erzeugt man dann durch jeweilige Messungen für eine größere Anzahl verschiedener Proben, beispielsweise indem man über einen bestimmten Zeitraum täglich eine Bierprobe aus der Produktion entnimmt und so vermisst, um den durch die verschiedenen eingangs genannten Einflüsse gegebenen Schwankungen der Probeneigenschaften Rechnung zu tragen. Wählt man eine ausreichende Anzahl von Proben über einen ausreichenden Zeitraum, z. B. indem man über einen oder mehrere Monate oder ein ganzes Jahr täglich ein oder mehrere Proben nimmt, kann man dadurch eine so große Anzahl verschiedener Proben erfassen, dass damit im Prinzip sämtliche Schwankungen, die in diesem Zeitraum vorkommen abgedeckt sind. Man erhält so eine Vielzahl von Messpunkten aus denen dann eine Datenmatrix gebildet wird, die sehr komplex sein kann. Daher geht man weiterhin so vor, dass man diesen komplexen multivariaten Datensatz nach der Methode der sogenannten Hauptkomponentenanalyse (PCA) reduziert, um diesen so in einen reduzierten Datensatz zu überführen, da der komplexe ursprüngliche Datensatz mathematisch nicht zu handhaben ist. Dieser reduzierte Datensatz dient dann als Modell für den Vergleich mit der Fremdprobe beziehungsweise fließt in dieses Modell ein.

Man kann beispielsweise so vorgehen, dass x Messwerte für eine Probe bei x verschiedenen Wellenlängen ein x-dimensionales Koordinatensystem liefern, welches dann durch Hauptkomponentenanalyse auf ein Koordinatensystem mit weniger Achsen, vorzugsweise mit nur zwei oder gegebenenfalls drei Achsen je nach Bedarf zurückgeführt wird. Ein solches zweidimensionales oder gegebenenfalls dreidimensionale Koordinatensystem kann man einer bildlichen Darstellung zugänglich machen. Wenn man beispielsweise die Messwerte in einem zweidimensionalen Koordinatensystem abbilden will, kann man diese beispielsweise auf dem Bildschirm eines transportablen Rechners (Laptop) am Messort in der Abbildung eines kartesischen Koordinatensystems sichtbar machen. Diese Messpunkte erscheinen in der Regel in der Darstellung als eine Punktwolke, bei der sich die einzelnen Messpunkte in der Fläche um ein Zentrum herum anordnen, wobei die Punktwolke in der Regel in der Dichte von dem Idealpunkt also von innen nach außen hin abnimmt. Einzelne Punkte im Peripheriebereich dieser Punktwolke entsprechen also Ausreißern, die auf nicht näher zu ergründende außergewöhnliche Schwankungen bei der Probennahme, Probenvorbereitung und/oder Messung einer individuellen Probe zurückgehen. Durch mathematisch-statistische Methoden wie vorzugsweise durch die oben genannte Hauptkomponentenanalyse eines multivariaten Datensatzes ist es aber in jedem Fall möglich auch eine Vielzahl von Messwerten der Proben der vorgegebenen Biermarke so zu reduzieren, dass sich eine zweidimensionale darstellbare Punktwolke mit endlicher Ausdehnung ergibt.

Vorzugsweise geht man dann weiter so vor, dass man die Fremdprobe untersucht, indem man diese spektroskopisch bei den gleichen Wellenlängen vermisst, wobei man gegebenenfalls mehrfache Messungen durchführt und eine Probenreihe von der Fremdprobe erzeugt, um Messfehler auszuschließen. Danach kann man vergleichen, ob diese Messwerte innerhalb des vorgegebenen Modells für die Biermarke des untersuchenden Herstellers liegen. Beispielsweise bei dem oben genannten Modell der Darstellung als Punktwolke in einem zweidimensionalen Koordinatensystem würde der oder die Messwerte für die Fremdprobe außerhalb der Punktwolke des Modells der vorgegebenen Biermarke liegen, wenn es sich um Bier einer anderen Biermarke handelt. Man kann aber anstelle der bildlichen Darstellung ebenso gut eine einfache tabellarische Darstellung wählen und die Probe "klassifizieren" indem man sie dem Modell zuordnet. Das Ergebnis erscheint dabei in einer ein- oder mehrspaltigen Tabelle mit einer der Fremdprobe zugeordneten Spalte beispielsweise als positiver oder negativer Vermerk, je nachdem ob die untersuchte Fremdprobe der vorgegebenen Biermarke entspricht oder nicht.

Ein Vorteil des erfindungsgemäßen Verfahrens liegt darin, dass die Untersuchung und Klassifizierung der Fremdprobe in der zuvor genannten Weise mit einer relativ einfachen Ausrüstung vor Ort durchführbar ist. Es handelt sich also um eine problemlos transportable Analysevorrichtung. Diese Vorrichtung zur analytischen Untersuchung einer Bierprobe im Rahmen der Anwendung des erfindungsgemäßen Verfahrens umfasst vorzugsweise ein Spektrometer für die Messung der Transmission oder Absorption im Infrarotbereich und/oder im sichtbaren Bereich, wenigstens eine eine Bierprobe aufnehmende Küvette, vorzugsweise eine Pumpe sowie Leitungen von der Pumpe zur Küvette und zum Spektrometer um die Probe durch die Küvette zu pumpen. Für die Auswertung kann man beispielsweise einen an das Messgerät angeschlossenen transportablen Rechner (Laptop) einsetzen, da die heute verfügbaren hochwertigen Laptops über eine ausreichende Rechenkapazität zur Durchführung der im Rahmen des erfindungsgemäßen Verfahrens notwendigen mathematisch-statistischen Auswertung verfügen, auch wenn es sich um komplexe Datensätze handelt. Auf diesem Rechner ist dann ein spezielles Datenverarbeitungsprogramm gespeichert, welches vorzugsweise zur Durchführung einer multivariaten Datenanalyse geeignet ist.

Auf diese Weise ist es möglich, eine Bierprobe vor Ort beispielsweise in einer Gaststätte zu nehmen und dort unmittelbar zu untersuchen und auszuwerten, wobei man zu einem Ergebnis gelangt, welches eine Zuordnung der untersuchten Bierprobe (Fremdprobe) zu einer vorgegebenen Biermarke mit einem sehr hohen Zuverlässigkeitsgrad ermöglicht.

Gemäß einer Weiterbildung der Erfindung lassen sich als flankierende Maßnahmen Farbmetrik und Sensorik in das erfindungsgemäße Konzept einbinden:

### Farbmetrik

Die Farbe des Bieres wird in EBC-Einheiten ausgedrückt und basiert auf dem Farbvergleich der Probe mit standardisierten EBC-Farbgläsern (EBC = European Brewery Convention).
Die Farbmetrik hingegen erlaubt die Bestimmung der Koordinaten eines Farbortes in einem international genormten Farbmaßsystem. Durch diese Koordinaten lässt sich jede Farbe eindeutig beschreiben.

### Sensorik

Sensorik ist die möglichst objektive Prüfung und Beschreibung von Lebensmitteln mit den menschlichen Sinnen durch geschulte Verkoster, wobei aus einzelnen subjektiven Ergebnissen eine objektive Aussage erarbeitet wird. Diese Aussage muss reproduzierbar sein.

Die in den Unteransprüchen genannten Merkmale betreffen bevorzugte Weiterbildungen des erfindungsgemäßen Untersuchungsverfahrens. Weitere Vorteile der Erfindung ergeben sich aus der nachfolgenden Detailbeschreibung.

Nachfolgend wird anhand von Ausführungsbeispielen unter Bezugnahme auf die beiliegenden Abbildungen die vorliegende Erfindung näher erläutert. Dabei zeigen,
- Fig. 1: eine schematisch vereinfachte Darstellung der Komponenten einer Vorrichtung zur analytischen Untersuchung einer Bierprobe im Rahmen des erfindungsgemäßen Verfahrens;
- Fig. 2: eine beispielhafte Darstellung von mehreren Transmissionsspektren verschiedener Biere, die nach dem erfindungsgemäßen Verfahren gemessen wurden;
- Fig. 3: eine Darstellung einer beispielhaften Datenmatrix eines Datensatzes für eine Probenreihe in vereinfachter Form;
- Fig. 4: eine vereinfachte bildliche Darstellung zur Erläuterung der Hauptkomponentenanalyse;
- Fig. 5: eine vereinfachte Darstellung zur Verdeutlichung der Klassifizierung einer fremden Bierprobe nach dem erfindungsgemäßen Verfahren.

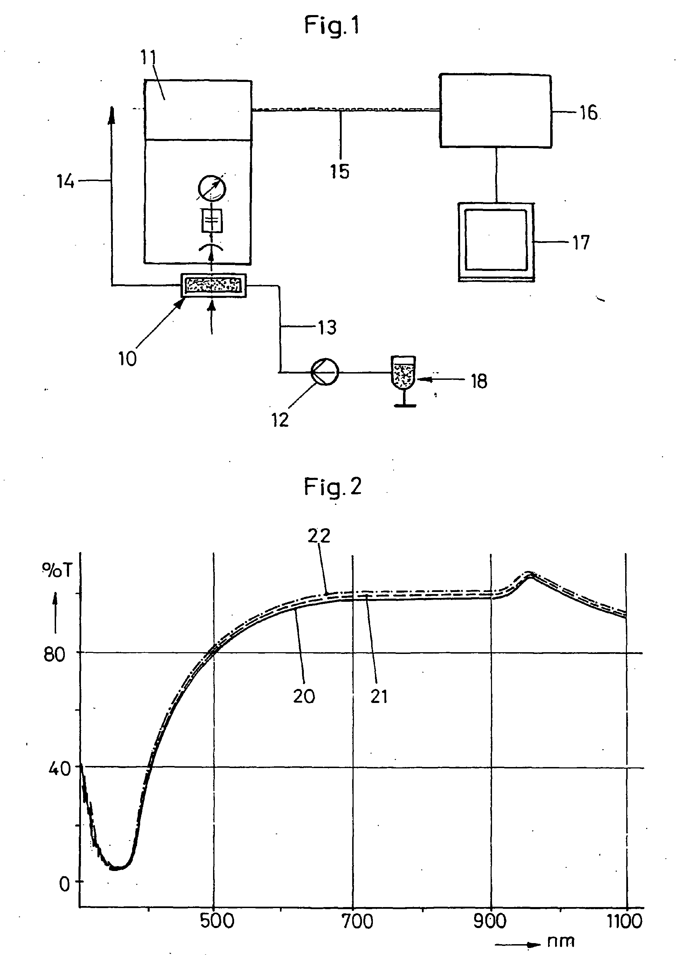

Zunächst wird auf Fig. 1 Bezug genommen. Von dem zu untersuchenden Bier wird in einem Probenbehältnis 18 eine Bierprobe genommen (Fremdprobe) und vorbereitet (z. B. entgast), die anschließend in der in Figur 1 dargestellten Messanordnung untersucht wird.

Die Darstellung zeigt in schematisch vereinfachter Form eine mögliche beispielhafte Vorrichtung, mittels der die analytische Untersuchung einer Bierprobe nach dem erfindungsgemäßen Verfahren vorgenommen werden kann. Die Vorrichtung besteht insgesamt aus wenigen Einzelbestandteilen mit vergleichsweise geringem Gewicht, so dass es sich um ein mobiles Labor handelt, welches von einer Person ohne großen Aufwand transportiert werden kann, so dass Untersuchungen vor Ort möglich sind, beispielsweise da, wo eine Bierprobe gezapft wird. Wie man aus Figur 1 erkennen kann, umfasst die Vorrichtung eine Küvette 10, die zugehörig ist zu einem Spektrometer 11, mittels dessen beispielsweise die Transmission der Probenflüssigkeit im nahen Infrarotbereich und im sichtbaren Bereich gemessen werden kann. Mittels einer Pumpe 12 wird durch die Leitung 13, 14 Probenflüssigkeit durch die Küvette 10 gepumpt und es wird die Transmission mittels des Spektrometers 11 gemessen. Über eine elektrische Verbindungsleitung und/oder Datenleitung 15 ist das Spektrometer 11 mit einem Rechner 16 verbunden. Mittels dieses Rechners 16 erfolgt die mathematisch/statistische Verarbeitung der in dem Spektrometer 11 gemessenen Messwerte. Als Ergebnis der Messungen erhält man eine Datenmatrix, die dann z. B. anhand der Hauptkomponentenanalyse auf eine Darstellung mit reduzierten Daten in einem vorzugsweise zwei- oder dreidimensionalen Koordinatensystem zurückgeführt wird. Das Modell für die vorgegebene Biermarke wird dann anhand der Daten erstellt.

Vor Ort wird in der Küvette 10 eine Fremdprobe eines Biers nicht bekannter Biermarke gemessen. Anhand einer Vielzahl von Messungen bei einer größeren Anzahl unterschiedlicher Wellenlängen wird für diese Fremdprobe mittels des Messgeräts 11 eine Datenmatrix im Rechner 16 erstellt. Die Daten für die Fremdprobe werden über die Datenleitung 15 an den Rechner 16 gegeben, dort verrechnet (Datenreduktion) und anschließend mit den zuvor gespeicherten, ebenso erstellten Daten des Modells verglichen. Dadurch kann eine Klassifizierung der gemessenen Fremdprobe, d. h., eine Zuordnung zu dem Modell einer vorher gemessenen und als Modell im Rechner 16 vorliegenden Biermarke erfolgen. Das Ergebnis der Klassifizierung kann beispielsweise unmittelbar auf einem Monitor 17, der mit dem Rechner 16 in Verbindung steht oder Teil des Rechners 16 ist, sichtbar gemacht werden. Zusätzlich oder alternativ dazu kann ein Ausdruck des Ergebnisses der Klassifizierung auf einem Drucker (nicht dargestellt) erfolgen.

Die Messergebnisse bei der erfindungsgemäßen Transmissionsmessung in einer Messanordnung der in Figur 1 wiedergegebenen Art und deren mathematisch statistische Auswertung werden nun nachfolgend unter Bezugnahme auf die weiteren Darstellungen Figur 2 bis Figur 5 näher erläutert. Dabei wird zunächst auf Figur 2 Bezug genommen. Diese Darstellung zeigt beispielhaft Transmissionsspektren verschiedener Bierproben, die mittels einer zuvor beschriebenen Messanordnung nach dem erfindungsgemäßen Verfahren gemessen wurden. In den Spektren nach Figur 2 ist die Transmission in Prozent auf der y-Achse dargestellt und aufgetragen gegen die Wellenlänge, wobei ein Wellenlängenbereich beginnend mit dem nahen UV bis zum nahen Infrarotbereich etwa bis 1.100 nm gemessen wurde. Dabei wurden viele Messpunkte bei unterschiedlichen Wellenlängen über den dargestellten Bereich gemessen und in der graphischen Darstellung gemäß Figur 2 als Spektralkurve wiedergegeben. Es sind dort die Transmissionsspektren von drei gemessenen Bierproben unterschiedlicher Biermarken wiedergegeben. Zur besseren Verdeutlichung ist das Spektrum 20 einer ersten Biermarke in ausgezogenen Linien dargestellt. Das Spektrum 21 einer zweiten Biermarke ist in gestrichelten Linien wiedergegeben. Das Spektrum 22, das für eine dritte Biermarke gemessen wurde, ist in strichpunktierten Linien dargestellt.

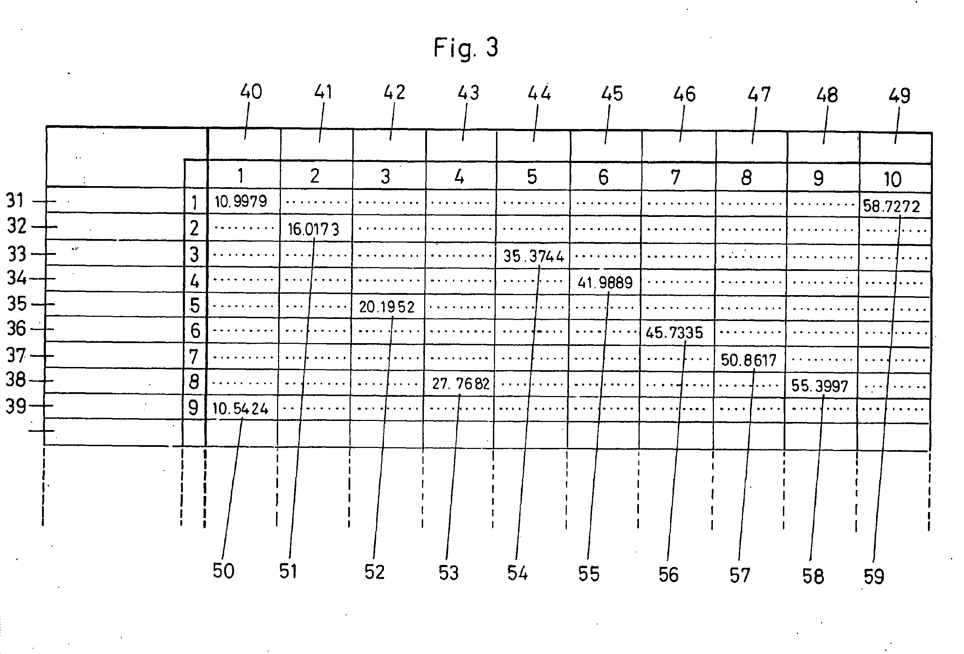

Die Darstellung gemäß Figur 2 zeigt, dass sich die drei beispielhaft wiedergegebenen Spektren 20, 21, 22 dreier verschiedener Biermarken sehr ähnlich sind. Abweichungen lassen sich zwar mit dem bloßen Auge erkennen. Nur anhand der Spektren ist es aber zunächst nicht möglich, eine zuverlässige qualitative Aussage zu treffen, die eine Klassifizierung, d. h. eine Zuordnung einer nach dem gleichen Verfahren gemessenen Fremdprobe zu einer der gemessenen Biermarken wie sie z. B. in Figur 2 dargestellt sind, ermöglicht. Hier setzt nun der zweite Teil des erfindungsgemäßen Verfahrens an, der darin besteht, dass aus den Messdaten ein Modell für die jeweilige Biermarke erstellt wird, welches dann anschließend die Zuordnung einer gemessenen Fremdprobe zu einem Modell einer bestimmten Biermarke zulässt. Dies wird nachfolgend unter Bezugnahme auf die Figuren 3 bis 5 näher erläutert.

Die Datenanalyse der Messdaten, die bei den spektroskopischen Transmissionsmessungen aufgenommen wurden, erfordert es, Korrelationen bzw. Beziehungsmuster zwischen den spektralen Messwerten und dem zugehörigen Biertypus zu suchen. Dies ist ein relativ aufwendiger Prozess. Zunächst erfordert die Bildung eines solchen Modells für eine bestimmte Biermarke die Erzeugung einer großen Anzahl von Messwerten. In einem beispielhaften erfindungsgemäßen Verfahren wurden pro Messung 1.840 Messwerte erzeugt, d. h., es wurde für eine Probe die Transmission bei 115 verschiedenen Wellenlängen in 16-facher Wiederholung gemessen. Auf diese Weise wurden 10 Proben untersucht. Um Messfehler auszuschließen erfolgte außerdem jeweils eine Doppelbestimmung. Damit ergeben sich bereits 36.800 Messwerte. Da das Bier aber einer zeitlichen Qualitätsschwankung unterliegt, auch wenn sich diese bei den modernen Produktionsmethoden in relativ engen Grenzen hält, sind Probenmessungen in regelmäßigen zeitlichen Abständen über einen längeren Zeitraum empfehlenswert. Beispielsweise ergeben sich Schwankungen in der Qualität durch unterschiedliche Chargen der eingesetzten Ausgangsstoffe wie Malz, gegebenenfalls Hopfen etc. Außerdem können Temperatur und Klimaschwankungen Einflüsse ausüben. Es empfiehlt sich daher Probenreihen jeweils für bestimmte festgelegte Zeiträume zu erstellen, um die Schwankungsbreiten zu ermitteln und dann anschließend bei der Erstellung des Modells entsprechend berücksichtigen zu können. Beispielsweise kann man jeweils über den Zeitraum eines Monats ein oder mehrmals täglich Proben der gleichen Biermarke ziehen und vermessen. Auf diese Weise kann man zunächst aus beispielsweise 30 Messungen ein Monatsmodell erstellen. Es empfiehlt sich dann weiterhin für jeden Monat beispielsweise über den Zeitraum eines Jahres ein eigenes Monatsmodell zu erstellen und daraus ein Jahresmodell zu bilden, was wiederum den Vorteil hat, dass man die einzelnen Monatsmodelle mit dem Jahresmodell vergleichen kann.

Die Vielzahl der dabei erhaltenen Messdaten kann man dann wieder z. B. durch Mittelwertbildung vereinfachen. Beispielsweise wurden in einem Fall für eine Bierprobe in einem Monat 560.000 Messwerte erzeugt und daraus wurden über 35.000 Mittelwerte gebildet, die als typische Merkmale zusammen mit den jeweils zugehörigen Proben in Form einer Datenmatrix angeordnet und dann mathematisch statistisch weiter ausgewertet wurden.

Hierzu bedient man sich im Rahmen des erfindungsgemäßen Verfahrens vorzugsweise der sogenannten multivariaten Datenanalyse. Wohingegen bei der traditionellen Datenanalyse nur ein oder zwei Variable gleichzeitig untersucht werden, erfolgt bei der multivariaten Datenanalyse die simultane Untersuchung aller Variablen großer Datensätze, die miteinander in Beziehung stehen, um ein Muster zu offenbaren. Das führt dann zu einem Modell, welches vorzugsweise über die Hauptkomponentenanalyse (PCA = principal component analysis) erstellt wird. Dabei wird der ursprüngliche Datensatz (beispielsweise 560.000 Datensätze) reduziert und es wird ein übersichtlicherer Datensatz erhalten. Eine mögliche verwendbare Software bei dieser multivariaten Datenanalyse bezeichnet man als "Unscrambler".

Zunächst werden die Daten in einer Datenmatrix dargestellt, in der beispielsweise vertikal verschiedene Proben angeordnet sind und horizontal die jeweiligen Wellenlängen, bei denen die Transmissionsmessungen erfolgt sind. Eine solche Datenmatrix ist stark vereinfacht in Figur 3 wiedergegeben. In der Spalte der Tabelle von Figur 3 ganz links sind untereinander verschiedene Probenbezeichnungen für gemessene Bierproben 31 bis 39 angegeben. Im Kopf der Tabelle sind über den jeweiligen Spalten die Wellenlängen 40 bis 49 wiedergegeben, bei denen durch Transmissionsmessung die Messwerte erzeugt wurden. Natürlich ist die Tabelle gemäß Figur 3 stark vereinfacht, d. h., tatsächlich werden wesentlich mehr Proben gemessen und wesentlich mehr unterschiedliche Wellenlängen, so dass in der Praxis die tatsächliche Tabelle viel umfangreicher ist. In die Tabelle gemäß Figur 3 sind beispielhaft einige Zahlenwerte 50 bis 59 eingetragen, die jeweils Messwerte darstellen, die für eine bestimmte Probe 31 bis 39 bei einer bestimmten Wellenlänge 40 bis 49 als Transmission gemessen wurden. Diese Messwerte 50 bis 59 werden numerisch so genau wie möglich, d. h., in der Regel mit mehreren Stellen hinter dem Komma angegeben.

Die Erstellung des Modells für eine bestimmte gemessene Biermarke erfolgt mittels der Hauptkomponentenanalyse, welche im Prinzip ein Projektionsverfahren ist. Die Hauptkomponenten bilden die Achsen eines transformierten Koordinatensystems, welches aus weniger Dimensionen besteht als das Original-Koordinatensystem. Die jeweiligen Projektionen für die einzelnen Proben auf die Hauptkomponenten ergeben Scores (Punkte), die in der graphischen Darstellung eine Mustererkennung ermöglichen.

Bei der Hauptkomponentenanalyse kann man im Prinzip jeden Messwertsatz für eine gemessene Probe als Punkt in einem mehrdimensionalen Raum betrachten. Jede Variable stellt eine Koordinatenachse dar. Die Koordinaten sind in einer Matrix tabelliert. Zur Reduzierung der Dimensionen dieser mehrdimensionalen Darstellung mit x Koordinatenachsen auf x - 1, x - 2 etc. Koordinatenachsen kann man, um dies anschaulich zu erläutern, sich vorstellen, dass z. B. eine Ebene wie eine Art Fenster in ein dreidimensionales Koordinatensystem der ursprünglichen Form hineingelegt wird und zwar derart, dass man die bestmögliche Annäherung erhält, d. h., man legt dieses Fenster so in den dreidimensionalen Raum, dass die Summe der Abweichungen von der Anordnung der Punkte in dem dreidimensionalen Raum bei der Reduzierung auf eine Ebene minimiert ist. Man erhält so eine um eine Dimension reduzierte Probenkarte, d. h., wenn man beispielsweise von einem dreidimensionalen Raum ausgeht eine zweidimensionale Annäherungsdarstellung. Dies gilt entsprechend auch für höhere Dimensionen, lässt sich dann allerdings nicht mehr mit vereinfachten Mitteln so gut veranschaulichen.

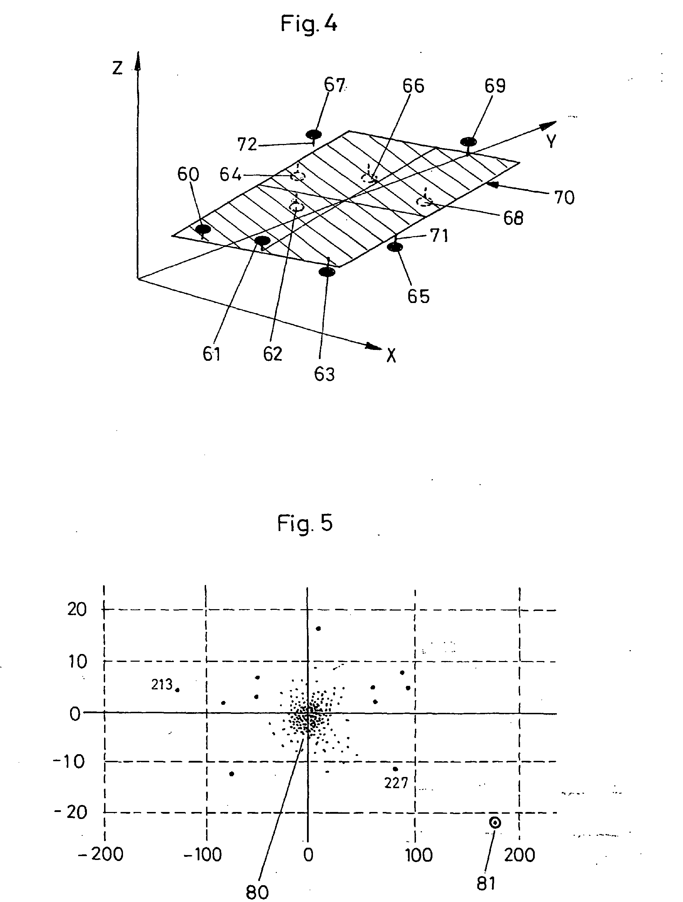

Diese Methode ist in Figur 4 bildlich erläutert. In der Darstellung ist ein Koordinatensystem mit drei Dimensionen wiedergegeben um einen dreidimensionalen Raum zu verdeutlichen. Das Koordinatensystem hat die Achsen x, y und z. Verschiedene Punkte für die jeweiligen Proben sind mit 60 bis 69 bezeichnet. Die Ebene oder das Fenster 70 wird nun so in den dreidimensionalen Raum hineingelegt, dass die Probenpunkte 60 bis 69 möglichst genau erfasst werden, d. h., dass die Summe der Abstände der einzelnen Proben minimal ist. Diese Abstände, die in der graphischen Darstellung gemäß Figur 4 als Nadeln dargestellt sind, sind mit 71, 72 bezeichnet. Den jeweiligen Abstand 71, 72 der einzelnen Probe zwischen ihrer tatsächlichen Position im dreidimensionalen Raum und ihrer in die zweidimensionale Ebene projizierten Position wird als Restvarianz bezeichnet und repräsentiert den durch die Vereinfachung erhaltenen Modellfehler.

Dieses Prinzip wurde anhand von Figur 4 nur beispielhaft als Reduzierung von einer dreidimensionalen auf eine zweidimensionale Darstellung erläutert. Tatsächlich handelt es sich natürlich in dem erfindungsgemäßen Verfahren um eine Transformation eines ursprünglichen Koordinatensystems mit wesentlich mehr Achsen. Beispielsweise wurde in einem erfindungsgemäßen Verfahren von einem Koordinatensystem mit 115 Achsen ausgegangen und dieses auf eine Hauptkomponentendarstellung mit nur zwei Achsen zurückgeführt.

Die Anordnung von Proben als Punkte in einer zweidimensionalen Darstellung lässt sich dann graphisch einfach darstellen und auf einem Bildschirm oder einem Ausdruck wiedergeben. Die Messwerte ergeben dabei z. B. einen Probenwolke, wie sie in Figur 5 dargestellt ist. Man sieht, dass die Punkte einer solchen Punktwolke (auch als Cluster bezeichnet) im Bereich um einen Mittelpunkt herum wesentlich dichter liegen, wohingegen die Punktdichte zum Rand hin abnimmt. Die Punktwolke ist insgesamt mit 80 bezeichnet, während Punkte, die einzelne Proben wiedergeben analog zu Figur 4 mit 60 bis 69 bezeichnet sind. Wenn man nun eine Fremdprobe durchmisst und diese klassifiziert und dabei festgestellt wird, dass diese Fremdprobe nicht zu der Biermarke gehört, zu der zuvor ein Modell erstellt wurde, dann ergibt sich in einem solchen Modell nach Figur 5, dass der Punkt für die Fremdprobe, der dort mit 81 bezeichnet ist, außerhalb der Punktwolke 80 der vorgegebenen Biermarke liegt.

## Patentansprüche

1. Verfahren zur analytischen Untersuchung einer Bierprobe, bei dem man zunächst durch spektroskopische Messungen einer Probenreihe verschiedener Proben mit jeweils mehreren Messwerten für jede Probe eine als Referenz dienende Datenmatrix erzeugt, die als Modell dient, und danach entsprechende Messwerte von einer zu untersuchenden Fremdprobe spektroskopisch erstellt werden und dann durch Vergleich geprüft wird, ob die Messwerte der Fremdprobe innerhalb des zuvor gewonnenen Modells liegen,
wobei die als Modell dienende Datenmatrix beim Hersteller durch spektroskopische Messungen einer Probenreihe verschiedener Proben einer vorgegebenen Biermarke erzeugt wird, **dadurch gekennzeichnet, dass** die zunächst als multivariater Datensatz vorliegende Datenmatrix durch Hauptkomponentenanalyse (PCA) in einen reduzierten Datensatz überführt wird, der als Modell für die zu vergleichende Fremdprobe dient, und dass die Fremdprobe mittels einer transportablen Analysevorrichtung vor Ort an einer Zapfstelle untersucht wird, um festzustellen, ob es sich bei dieser um Bier einer bestimmten vorgegebenen Biermarke handelt, für die zuvor das Modell erstellt wurde.

2. Verfahren zur analytischen Untersuchung einer Bierprobe nach Anspruch 1, **dadurch gekennzeichnet, dass** als spektroskopische Messung mindestens die Transmission oder Absorption der Proben im Infrarotbereich gemessen wird.

3. Verfahren zur analytischen Untersuchung nach Anspruch 1 oder 2, **dadurch gekennzeichnet, dass** die Transmission oder Absorption der Proben im nahen Infrarotbereich und im sichtbaren Bereich gemessen wird.

4. Verfahren zur analytischen Untersuchung einer Bierprobe nach einem der Ansprüche 1 bis 3, **dadurch gekennzeichnet, dass** die als Referenz dienende Datenmatrix durch Messung der Transmission oder Absorption bei einer größeren Anzahl unterschiedlicher Wellenlängen für jeweils eine größere Anzahl verschiedener Proben der vorgegebenen Biermarke erzeugt wird.

5. Verfahren zur analytischen Untersuchung einer Bierprobe nach einem der Ansprüche 1 bis 4, **dadurch gekennzeichnet, dass** x Messwerte für eine Probe bei x verschiedenen Wellenlängen ein x-dimensionales Koordinatensystem liefern, welches durch Hauptkomponentenanalyse auf ein Koordinatensystem mit nur zwei, drei oder gegebenenfalls n Achsen (Hauptkomponenten) zurückgeführt wird.

6. Verfahren zur analytischen Untersuchung einer Bierprobe nach einem der Ansprüche 1 bis 5, **dadurch gekennzeichnet, dass** durch Untersuchung mehrerer Probenreihen mehrerer verschiedener vorgegebener Biermarken für diese jeweils die als Referenz dienenden Datenmatrizes erzeugt werden die mehrere den jeweiligen Biermarken entsprechende Modelle bilden und nach Erstellung entsprechender Messwerte für die Fremdprobe diese durch Zuordnen zu einem der Modelle klassifiziert wird.

## Claims

1. Method for analytical examination of a beer sample, in which a data matrix which is used as a reference and is used as a model is first of all produced by spectroscopic measurements of a sample series of different samples, each having a plurality of measured values for each sample, and corresponding measured values are then created spectroscopically from an external sample to be examined, and a check is then carried out by comparison to determine whether the measured values of the external sample are within the previously obtained model,
wherein the data matrix which is used as a model is produced at the manufacturer by spectroscopic measurements of a sample series of different samples of a predetermined beer brand,
**characterized in that** the data matrix, which is initially in the form of a multivariate data record, is changed by primary component analysis (PCA) to a reduced data record which is used as a model for the external sample to be compared, and **in that** the external sample is examined in situ at a tapping point by means of a transportable analysis apparatus, in order to determine whether this is beer of a specific predetermined beer brand for which the model was previously created.

2. Method for analytical examination of a beer sample according to claim 1, **characterized in that** at least the transmission or absorption of the samples is measured in the infrared range as a spectroscopic measurement.

3. Method for analytical examination according to claim 1 or 2, **characterized in that** the transmission or absorption of the samples is measured in the near infrared range or in the visible range.

4. Method for analytical examination of a beer sample according to one of claims 1 to 3, **characterized in that** the data matrix which is used as a reference is produced by measurement of the transmission or absorption at a relatively large number of different wavelengths in each case for a relatively large number of different samples of the predetermined beer brand.

5. Method for analytical examination of a beer sample according to one of claims 1 to 4, **characterized in that** x measured values for a sample produce an x-dimensional coordinate system at x different wavelengths, which coordinate system is fed back by means of primary component analysis to a coordinate system with only two, three or possibly n axes (primary components).

6. Method for analytical examination of a beer sample according to one of claims 1 to 5, **characterized in that** the data matrices which are used as a reference are in each case produced for a plurality of different predetermined beer brands by examination of a plurality of sample series of these beer brands, which data matrices form a plurality of models which correspond to the respective beer brands and, after creation of corresponding measured values for the external sample, the latter is classified by association with one of the models.

## Revendications

1. Procédé pour l'analyse d'un échantillon de bière, dans lequel on génère tout d'abord une matrice de données servant de référence au moyen de mesures spectroscopiques d'une série d'échantillons de différents échantillons avec plusieurs valeurs de mesures pour chaque échantillon respectif, cette matrice de données servant de modèle, et on établit ensuite par spectroscopie des valeurs de mesure correspondantes à partir d'un échantillon étranger à examiner et on vérifie alors par comparaison si les valeurs de mesure de l'échantillon étranger se situent à l'intérieur du modèle élaboré précédemment,
dans lequel la matrice de données servant de modèle est générée par le fabricant au moyen de mesures spectroscopiques d'une série d'échantillons de différents échantillons d'une marque de bière définie,
**caractérisé en ce qu'**on transforme la matrice de données existant tout d'abord sous la forme d'une structure de données de variables aléatoires multiples par analyse des composantes essentielles (PCA) en une structure de données réduite, servant de modèle à l'échantillon étranger à comparer, et **en ce qu'**on analyse l'échantillon étranger sur place à un point de tirage au moyen d'un dispositif d'analyse transportable, pour constater s'il s'agit là d'une bière d'une marque de bière spécifique définie, pour laquelle on a établi le modèle antérieurement.

2. Procédé pour l'analyse d'un échantillon de bière selon la revendication 1, **caractérisé en ce que** pour la mesure spectroscopique, on mesure au moins la transmission ou l'absorption des échantillons dans le domaine infrarouge.

3. Procédé pour l'analyse selon la revendication 1 ou 2, **caractérisé en ce qu'**on mesure la transmission ou l'absorption des échantillons dans le domaine infrarouge proche et dans le domaine visible.

4. Procédé pour l'analyse d'un échantillon de bière selon une des revendications 1 à 3, **caractérisé en ce qu'**on génère tout d'abord la matrice de données servant de référence par la mesure de la transmission ou de l'absorption à un plus grand nombre de différentes longueurs d'ondes respectivement pour un plus grand nombre d'échantillons différents de la marque de bière définie.

5. Procédé pour l'analyse d'un échantillon de bière selon une des revendications 1 à 4, **caractérisé en ce que** x valeurs de mesure d'un échantillon à x longueurs d'ondes différentes fournissent un système de coordonnées à x dimensions, qui est ramené par analyse des composantes essentielles à un système de coordonnées à seulement deux, trois ou éventuellement n axes (composantes principales).

6. Procédé pour l'analyse d'un échantillon de bière selon une des revendications 1 à 5, **caractérisé en ce que** l'analyse de plusieurs séries d'échantillons de plusieurs marques différentes de bière définies génère pour celles-ci les matrices de données servant respectivement de référence, qui forment plusieurs modèles correspondants aux marques de bière respectives et qui, après établissement des valeurs de mesure correspondantes de l'échantillon étranger, classifient celui-ci en l'attribuant à un des modèles.
